# FASCICULE DE BREVET EUROPEEN

(11) **EP 0 879 414 B1**
(45) Date de publication et mention de la délivrance du brevet: **20.08.2003**
(21) Numéro de dépôt: 97900237.5
(22) Date de dépôt: 07.01.1997
(51) Int. Cl.: G01N 33/483, G01N 33/50, C12Q 1/02

(54) **PROCEDE POUR L'EVALUATION DE LA MIGRATION DES KERATINOCYTES**
VERFAHREN ZUR BEWERTUNG DER KERATINOCYTEMIGRATION
METHOD FOR EVALUATING KERATINOCYTE MIGRATION

(30) Priorité: 08.01.1996 FR 9600121
(43) Date de publication de la demande: 25.11.1998
(73) Titulaire: ASSOCIATION POUR L'ESSOR DE LA TRANSFUSION SANGUINE DANS LA REGION DU NORD, 59000 Lille (FR); INSTITUT NATIONAL DE LA SANTE ET DE LA RECHERCHE MEDICALE (INSERM), 75013 Paris (FR)
(72) Inventeur: RONFARD, Vincent, Canton MA 0201 (US); BARRANDON, Yann, F-75011 Paris (FR)
(74) Mandataire: Lhuillier, René
(86) Numéro de dépôt international: FR9700020
(87) Numéro de publication internationale: WO97025617

(56) Documents cités:
- EP-A- 0 373 044
- US-A- 4 359 527
- JOURNAL OF INVESTIGATIVE DERMATOLOGY, vol. 100, no. 5, 1 Mai 1993, NEW YORK, NY, pages 633-639, XP000195880 ANDO ET AL: "EPIDERMAL GROWTH FACTOR AND INSULIN-LIKE GROWTH FACTOR I ENHANCE KERATINOCYTE MIGRATION" cité dans la demande
- CELL ADHESION AND COMMUNICATION, vol. 2, 1994, pages 299-308, XP000196368 DONALDSON ET AL.: "Further studies on the interaction of migrating keratinocytes with fibrinogen"

## Description

L'invention concerne un procédé pour la mise en évidence et la mesure de la migration des kératinocytes dans un substrat à base de fibrine. La reproductibilité du test permet de l'utiliser pour l'évaluation de diverses conditions biologiques, chimiques ou physiques sur la capacité migratoire des cellules.

Les kératinocytes représentent plus de 90 % des cellules de l'épiderme humain. Le renouvellement permanent de l'épiderme est assuré par les kératinocytes situés dans la couche basale. Dès qu'ils ont quitté celle-ci, ils perdent la capacité de se diviser et ils se différencient pour former la couche cornée. Au contact de glycoprotéines de la membrane basale, les kératinocytes établissent des structures d'adhérence forte (les hémidesmosomes) qui les rendent immobiles latéralement. Par contre en cas de réépithélialisation de plaies, les kératinocytes basaux entrent en contact avec d'autres molécules comme la fibronectine, le collagène, la thrombospondine... qui induisent une importante mobilité latérale de ces kératinocytes et favorisent ainsi la cicatrisation.

Différentes techniques sont utilisées pour étudier la migration cellulaire : - l'observation au microscope couplé à une caméra vidéo permet de suivre le déplacement des cellules de manière continue (le procédé est coûteux et nécessite un matériel complexe comprenant un mini-incubateur à CO₂ placé sous un microscope équipé d'une caméra).
- la mesure de la migration à partir d'explants insérés dans des gouttes d'agarose ("agarose drop explant") ou dans des "chambres de Boyden" (migration au travers de membranes poreuses) ne permettent qu'une évaluation collective de migration et de prolifération cellulaires.
- le déplacement individuel de cellules a été observé sur des lamelles de verre recouvertes de particules d'or colloïdal, avec des cellules 3T3 (G.Albrecht-Buehler - Cell 11, 1977, 395) : les cellules migrent en ingérant les particules d'or, ce qui laisse une trace visible de leur déplacement (après fixation au formaldéhyde, les cellules sont photographiées au microscope en champ noir ; les traces de migrations apparaissent en noir dans le champ brillant de particules d'or) ; ce test a reçu le nom de "phagokinetic track assay". Il a pu être reproduit avec des kératinocytes à condition de recouvrir la couche d'or colloïdal d'une matrice de collagène IV (Y.Ando et P.J.Jensen J.Invest Dermmat.100,1993,633) . Ce test permet d'étudier l'influence de divers facteurs de croissance, cytokines ou hormones sur la migration des cellules.
   La Demanderesse a mis au point un nouveau procédé d'observation de la migration des kératinocytes dans des conditions plus physiologiques que celles décrites précédemment (c'est à dire sans phagocytose de particules d'or) et plus particulièrement en situation proche de celle de la réépithélialisation d'une blessure.
   Quand une blessure cutanée se produit, le premier événement qui intervient est la formation d'un caillot de fibrine dont la fonction est d'arrêter le saignement. Ensuite les kératinocytes migrent sous ce caillot, à partir de la couche basale des bords de la blessure.
   Par analogie avec ce mécanisme, la Demanderesse a déjà décrit (brevets FR 2 639 958 et EP 0 373 044) l'utilisation d'un support à base de fibrine pour la culture de kératinocytes, ladite culture étant directement utilisable comme greffon. Dans ce système, la présence d'une antiprotéase, en particulier l'aprotinine, est indispensable pour empêcher la dégradation du substrat de fibrine par les protéases des cellules et du sérum.
   La Demanderesse a maintenant montré que le même substrat, mais dépourvu d'aprotinine, permet d'observer la migration des kératinocytes, inoculés à faible densité cellulaire (environ 10³ cells/cm²) en présence d'une faible quantité de sérum.
   Dans des conditions identiques, les fibroblastes du derme humain ne présentent aucune mobilité.
   Le comportement des kératinocytes n'est pas homogène
- une fraction des cellules (65 %) est immobile ou présente un déplacement inférieur au diamètre d'une cellule,
- l'autre fraction des cellules (35 %) se déplace en dégradant le substrat de fibrine et en y laissant l'empreinte de son passage sous forme de galeries cylindriques ou hélicoïdales. La taille de ces galeries est assez constante : en moyenne 19-20 µm de longueur et un peu plus d'une cellule de diamètre (12-15 µm) par heure.

Les kératinocytes en migration ont un aspect sphérique et leur mouvement comprend une rotation, soit dextrogyre soit lévogyre (en moyenne 50 % de chaque type), comme on peut l'observer sur les photographies des empreintes dans la fibrine.

En absence de sérum, les kératinocytes ne migrent pas. Le sérum apporte probablement la quantité de plasminogène (qui sera activé en plasmine) nécessaire à la dégradation de la fibrine en présence des protéases cellulaires.

La Demanderesse a tiré parti de ce phénomène pour mettre au point un procédé d'évaluation de la migration des kératinocytes à la fois simple, rapide et ne nécessitant pas d'équipement sophistiqué, et qui permet ainsi d'étudier l'effet de divers facteurs ajoutés au milieu de culture des cellules ainsi que de diverses conditions physiques, biologiques ou chimiques. Ce test est ainsi particulièrement avantageux pour des études à but dermatologique ou cosmétologique.

Le procédé selon l'invention comprend :
- l'utilisation de plaques multipuits pour cultures de cellules ou de lamelles pour observation microscopique, recouvertes d'une fine couche transparente de fibrine réticulée en présence de thrombine ;
- l'utilisation, pour former ladite couche de fibrine, d'un concentré de fibrinogène approprié pour former une colle biologique, dépourvu d'antiprotéase exogène ;
- l'inoculation des kératinocytes à très faible densité cellulaire, de préférence de l'ordre de 10³ cellules par cm² ;
- l'incubation en milieu de culture pour kératinocytes additionné de 1 à 10 % de sérum, en absence d'antiprotéase exogène (en particulier en absence d'aprotinine), pendant 20 à 24 heures à 37°C ;
- l'élimination du milieu de culture et la fixation des kératinocytes au formaldéhyde ;
- l'observation au microscope, éventuellement après photographie, d'un nombre de champs statistiquement significatif et le comptage des kératinocytes ayant migré et l'évaluation de la longueur de l'empreinte de leur migration.

Le procédé selon l'invention peut être utilisé avantageusement pour étudier l'effet sur la migration des kératinocytes :
- de facteurs physiologiques comme les hormones, les cytokines ou toute molécule susceptible d'en influencer le métabolisme ;
- de molécules d'intérêt thérapeutique du type des facteurs favorisant la cicatrisation ;
- de molécules d'intérêt cosmétologique comme des facteurs de protection contre les rayonnements, la température ou toute condition physique ou chimique délétère pour l'épiderme.

Le procédé selon l'invention peut aussi être utilisé pour étudier l'effet de conditions physiques, chimiques ou biologiques (comme le contact avec des cellules nourricières) susceptibles d'affecter le métabolisme des kératinocytes.

Le procédé selon l'invention peut encore être utilisé pour étudier l'état biologique des kératinocytes afin de diagnostiquer tout état pathologique comme une déficience en facteur de cicatrisation ou un état de transformation néoplasique.

La mise en oeuvre du procédé pour l'évaluation de différentes conditions de culture sera mieux comprise dans les exemples qui suivent. Ceux-ci ne constituent pas une limitation de la portée de l'invention.

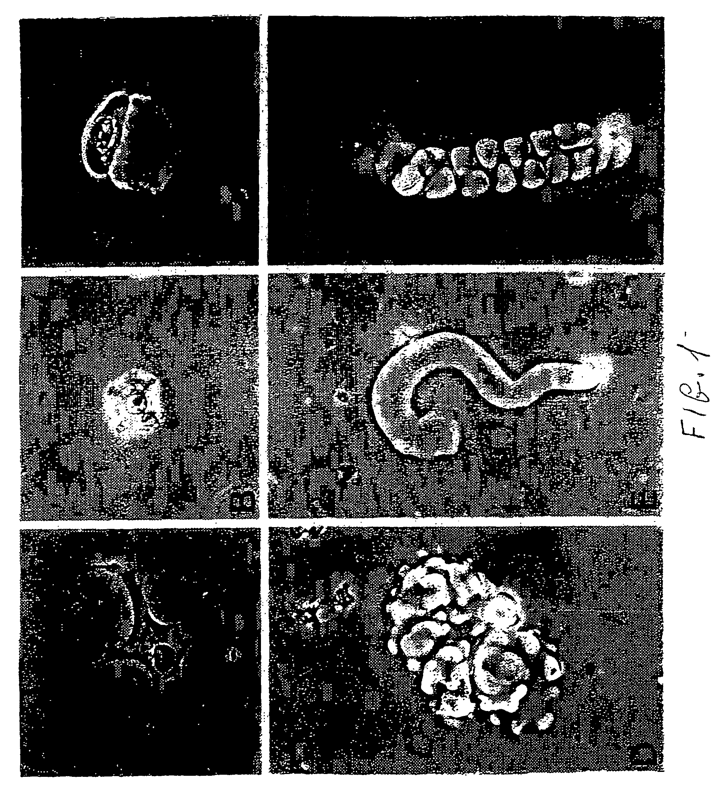

La figure 1 est décrite dans l'exemple I,3.

### Exemple I - Mise au point du test. Choix des cellules.

### 1. Préparation du substrat.

Le substrat de fibrine réticulée est préparé à partir d'une préparation commerciale de colle biologique (Biocol®-Thrombine humaine fournie par Biotransfusion-Lille-France). Le concentré de fibrinogène lyophylisé de cette préparation est un concentré de protéines plasmatiques humaines comprenant plus de 70 % de fibrinogène, du Facteur XIII et de la fibronectine (EP 0 305 243). La quantité lyophilisée dans un flacon de 5 ml est reconstituée dans 10,8 ml d'eau bidistillée stérile. La thrombine humaine lyophilisée, dosée à 500 U NIH/ml est diluée avec du sérum physiologique pour obtenir une concentration finale de 5 à 10 U/ml. Les deux composants sont reconstitués en moins de 15 minutes à température ambiance ; ils sont injectés simultanément sur le fond des puits de culture (plaque multipuits pour culture cellulaire, distribuée par exemple par Nunc®) à l'aide du mélangeur fourni dans la trousse de Biocol®-thrombine. Chaque puits de culture de 35 mm de diamètre reçoit 0,25 ml de chacun des deux constituants. En quelques minutes il se forme un réseau uniforme de fibrine transparente, prête à l'emploi, d'une épaisseur moyenne de 50 à 100 µm.

### 2. Cellules.

Les kératinocytes proviennent d'une culture fraîchement préétablie. Des kératinocytes provenant de différentes parties du corps (16 prélèvements) ont été utilisés entre les passages 2 et 7, avec des résultats semblables, indépendamment de l'âge du donneur. Par contre des cellules primaires (fraîchement isolées et non établies en culture) ne présentent pas de capacité de migration dans la fibrine.

Les cultures de kératinocytes trypsinisées et diluées en milieu de culture sont ensemencées à une densité de 10³ cellules par puits de 35 mm de diamètre. Elles sont incubées à 37°C en milieu de culture contenant 10 % de sérum foetal bovin, en absence d'antiprotéase exogène et en absence de couche nourricière de cellules 3T3.

### 3. Observation de la migration des cellules.

Après 24 heures d'incubation à 37°C, le surnageant des cultures est jeté et les cellules adhérentes au substrat sont fixées avec une solution à 3,7 % de formaldéhyde pour être observées au microscope. On utilise un objectif de grossissement 10. Des mesures statistiquement valables sont obtenues par observation de 18 champs microscopiques pris au hasard (6 champs par boîte et 3 boîtes par condition expérimentale, c'est-à-dire pour un point sur un graphique).

Quand on applique le test à l'évaluation du rôle d'un facteur de croissance (ou toute autre molécule), celui-ci est additionné dans le milieu de culture des cellules, sans période de préincubation.

Un résultat typique après 24 heures d'incubation est présenté sur la figure 1 :
la photo A montre un fibroblaste humain
les photos B et C montrent un kératinocyte immobile et un très peu mobile
les photos D à F montrent des kératinocytes très mobiles.
En particulier la photo F montre l'empreinte hélicoïdale régulière laissée dans la fibrine par la migration de la cellule.

### Exemple II - Reproductibilité du test.

4 lots de Biocol® ont été utilisés et les kératinocytes d'une culture préétablie ont été ensemencés, comme décrit plus haut, et incubés à 37°C pendant 24 heures. La proportion de cellules mobiles est similaire pour les 4 lots :

| lots de Biocol® | % de cellules mobiles |
|---|---|
| 0730 | 23 |
| 0960 | 31 |
| 0830 | 28 |
| 0870 | 30 |
| | moyenne : 28 % |

Avec un type cellulaire choisi la reproductibilité du test est excellente et permettra donc d'analyser l'influence de divers facteurs physiques ou chimiques.

### Exemple III - Variabilité de la migration cellulaire

Différents types de kératinocytes migrent plus ou moins fort, mais au sein d'une lignée le pourcentage de cellules qui migrent est bien reproductible.

3 lignées de kératinocytes ont été testées au quatrième et cinquième passages en culture, dans les conditions décrites plus haut.

| Culture (code de laboratoire) | % de cellules mobiles (moyenne de 7 expériences) |
|---|---|
| YF29 V | 44 |
| YE1 IV | 31 |
| AFL1 IV | 14 |

De manière surprenante, on a observé que des kératinocytes immortalisés ou néoplasiques (provenant de carcinomes à cellules squameuses) ont pratiquement perdu leur capacité migratoire. Cette observation peut être corrélée à la faible tendance métastasique de ces cellules. Cette observation souligne l'intérêt diagnostic potentiel du test selon l'invention.

### Exemple IV - Etat de différenciation des kératinocytes

Les kératinocytes ont été ensemencés sur le substrat de fibrine à partir d'une même culture établie depuis 5, 6, 7 et 10 jours. On constate que la mobilité des kératinocytes est inversement proportionnelle à l'âge de la culture

| jours de culture de YF29 V | % de cellules mobiles |
|---|---|
| 5 | 50 |
| 6 | 35 |
| 7 | 18 |
| 10 | 5 |

D'autre part, on observe que des kératinocytes fraîchement isolés ne migrent pas et ne dégradent pratiquement pas la fibrine pendant les 2 premiers jours suivant la mise en culture.

48 heures plus tard leur migration est de 6µm/heure et après quelques passages en culture, leur migration est de 48µm/heure. Ce résultat coïncide avec l'observation, in vivo, du début de la cicatrisation d'une blessure après plus d'un jour, les cellules devant passer du stade "établies" à un stade "hyperprolifératif".

### Exemple V - Mise en évidence de l'effet des facteurs de croissance

Différentes souches de kératinocytes ont été ensemencées sur le substrat de fibrine avec ou sans apport d'EGF ("epidermal growth factor") à 10 ng/ml. La proportion moyenne de cellules mobiles est doublée en présence d'EGF (movenne de 3 expériences) :

| | % de cellules mobiles | |
|---|---|---|
| souches | sans EGF | avec EGF |
| YF 29 | 34 | 69 |
| YE 1 | 26 | 47 |
| AFL 1 | 14 | 51 |

De plus la proportion de cellules mobiles augmente avec la concentration d'EGF.

Dans un test réalisé avec des kératinocytes provenant d'une culture jeune, non confluente, on a observé le résultat suivant :

| concentration en EGF (ng/ml) | % de kératinocytes mobiles |
|---|---|
| 0 | 21 |
| 0,1 | 43 |
| 1 | 57 |
| 10 | 64 |
| 100 | 68 |

De plus l'EGF augmente la vitesse de migration parce que la longueur des empreintes dans la fibrine est significativement plus longue.

### exemple VI - Mise en évidence d'effets négatifs sur la migration.

Le même test permet d'étudier l'effet inhibiteur de certains facteurs sur la migration des kératinocytes.

A titre d'exemple on a mesuré la migration (en présence et en absence d'EGF) en présence de 3 concentrations de thrombine :

| thrombine UI/ml | % de kératinocytes mobiles | |
|---|---|---|
| | sans EGF | avec EGF |
| 0 | 36 | 54 |
| 10 | 28 | 47 |
| 30 | 11 | 24 |
| 100 | 12 | 24 |

La thrombine inhibe la mobilité ; cet effet est moins marqué en présence d'EGF.

## Revendications

1. Procédé pour l'évaluation de la migration des kératinocytes, **caractérisé en ce qu'**il comprend l'observation au microscope de l'empreinte créée par la migration cellulaire dans un substrat à base de fibrine réticulée, après inoculation des kératinocytes à faible densité cellulaire et incubation en milieu de culture additionné de sérum et dépourvu d'antiprotéase exogène.

2. Procédé selon la revendication 1, **caractérisé en ce qu'**il comprend l'utilisation, pour former la couche de fibrine, d'un concentré de fibrinogène d'origine plasmatique approprié pour former une colle biologique en présence de thrombine, dépourvu d'antiprotéase exogène.

3. Procédé selon la revendication 1 ou 2, **caractérisé en ce qu'**il comprend
- l'utilisation de plaques multipuits pour cultures de cellules ou de lamelles pour observation microscopique, recouvertes d'une fine couche transparente de fibrine réticulée en présence de thrombine ;
- l'inoculation des kératinocytes à très faible densité cellulaire, de préférence de l'ordre de 10³ cellules par cm² ;
- l'incubation en milieu de culture pour kératinocytes additionné de 1 à 10 % de sérum, en absence d'antiprotéase exogène (en particulier en absence d'aprotinine), pendant 20 à 24 heures à 37°C ;
- l'élimination du milieu de culture et la fixation des kératinocytes au formaldéhyde ;
- l'observation au microscope, éventuellement après photographie, d'un nombre de champs statistiquement significatif et le comptage des kératinocytes ayant migré et l'évaluation de la longueur de l'empreinte de leur migration.

4. Utilisation du procédé selon l'une quelconque des revendications 1 à 3 pour étudier l'effet sur la migration des kératinocytes de facteurs physiologiques comme les hormones, les cytokines ou toute molécule susceptible d'en influencer le métabolisme.

5. Utilisation du procédé selon l'une quelconque des revendications 1 à 3 pour étudier l'effet sur la migration des kératinocytes de molécules d'intérêt thérapeutique du type des facteurs favorisant la cicatrisation.

6. Utilisation du procédé selon l'une quelconque des revendications 1 à 3 pour étudier l'effet sur la migration des kératinocytes de molécules d'intérêt cosmétologique comme des facteurs de protection contre les rayonnements, la température ou toute condition physique ou chimique délétère pour l'épiderme.

7. Utilisation du procédé selon l'une quelconque des revendications 1 à 3 pour étudier l'effet de conditions physiques chimiques ou biologiques susceptibles d'affecter le métabolisme des kératinocytes.

8. Utilisation du procédé selon l'une quelconque des revendications 1 à 3 pour étudier l'état biologique des kératinocytes afin de diagnostiquer tout état pathologique comme une déficience en facteur de cicatrisation ou un état de transformation néoplasique.

## Patentansprüche

1. Verfahren zur Bewertung der Keratinozytenmigration, **dadurch gekennzeichnet, dass** es die Beobachtung der Spur, die durch die Zellmigration in einem Substrat auf der Basis von vemetztem Fibrin nach der Einimpfung von Keratinozyten mit geringer Zelldichte und der Inkubation in einem Kulturmedium, das mit Serum versetzt ist und von exogener Antiprotease frei ist, erzeugt wird, unter einem Mikroskop umfasst.

2. Verfahren nach Anspruch 1, **dadurch gekennzeichnet, dass** es zur Bildung der Fibrinschicht die Verwendung eines Konzentrats von Fibrinogen plasmatischer Herkunft, das zur Bildung eines biologischen Klebstoffs in Gegenwart von Thrombin geeignet ist und von exogener Antiprotease frei ist, umfasst.

3. Verfahren nach Anspruch 1 oder 2, **dadurch gekennzeichnet, dass** es umfasst
die Verwendung von Multiwell-Zellkulturplatten oder von Deckgläsem zur mikroskopischen Beobachtung, die überzogen sind mit einer feinen transparenten Schicht von in Gegenwart von Thrombin vemetztem Fibrin;
die Einimpfung von Keratinozyten mit sehr geringer Zelldichte, vorzugsweise in der Größenordnung von 10³ Zellen pro cm²;
die Inkubation in Kulturmedium für Keratinozyten, das mit 1 bis 10 % Serum versetzt ist, in Abwesenheit von exogener Antiprotease (insbesondere in Abwesenheit von Aprotinin), während 20 bis 24 Stunden bei 37 °C;
die Entfernung des Kulturmediums und die Fixierung der Keratinozyten mit Formaldehyd;
die Beobachtung einer statistisch signifikanten Anzahl von Feldern unter einem Mikroskop, gegebenenfalls nach einer Fotografie, und das Zählen der Keratinozyten, die gewandert sind, und die Bewertung der Länge der Spur ihrer Migration.

4. Verwendung des Verfahrens nach einem der Ansprüche 1 bis 3 zum Untersuchen der Wirkung von physiologischen Faktoren wie Hormonen, Zytokinen oder jedem Molekül, das im Stande ist, den Metabolismus zu beeinflussen, auf die Keratinozytenmigration.

5. Verwendung des Verfahrens nach einem der Ansprüche 1 bis 3 zum Untersuchen der Wirkung von Molekülen von therapeutischem Interesse von der Art der Faktoren, welche die Wundheilung begünstigen, auf die Keratinozytenmigration.

6. Verwendung des Verfahrens nach einem der Ansprüche 1 bis 3 zum Untersuchen der Wirkung von Molekülen von kosmetologischem Interesse wie etwa den Faktoren, die Schutz gegen Strahlung, die Temperatur oder jeden physikalischen oder chemischen Zustand, der für die Epidermis schädlich ist, bieten, auf die Keratinozytenmigration.

7. Verwendung des Verfahrens nach einem der Ansprüche 1 bis 3 zum Untersuchen der Wirkung von physikalischen, chemischen oder biologischen Zuständen, die im Stande sind, den Metabolismus von Keratinozyten zu beeinflussen.

8. Verwendung des Verfahrens nach einem der Ansprüche 1 bis 3 zum Untersuchen des biologischen Zustandes von Keratinozyten, um jeden pathologischen Zustand wie etwa einen Mangel an Wundheilungsfaktor oder einen Zustand der neoplastischen Transformation zu diagnostizieren.

## Claims

1. Method of evaluating the migration of keratinocytes, **characterised in that** it comprises observation, under a microscope, of the imprint produced by cell migration in a substrate based on crosslinked fibrin, following inoculation of keratinocytes at low cell density and incubation in culture medium to which serum has been added and which is free from exogenous antiprotease.

2. Method according to claim 1, **characterised in that** it comprises using, for formation of the fibrin layer, a concentrate of fibrinogen of plasma origin which is suitable for formation of a biological glue in the presence of thrombin and free from exogenous antiprotease.

3. Method according to claim 1 or 2, **characterised in that** it comprises
- use of multiwell plates for cell cultures or slides for microscope observation, covered by a thin transparent layer of crosslinked fibrin in the presence of thrombin;
- inoculation of keratinocytes at very low cell density, preferably of the order of 10³ cells per cm²;
- incubation in culture medium for keratinocytes to which there has been added 1 to 10 % serum, in the absence of exogenous antiprotease (especially in the absence of aprotinin), for 20 to 24 hours at 37°C;
- removal of the culture medium and fixing of the keratinocytes using formaldehyde;
- observation under a microscope, optionally after photography, of a statistically significant number of fields, and counting of the keratinocytes having migrated, and evaluation of the length of the imprint of their migration.

4. Use of the method according to any one of claims 1 to 3 in studying the effect, on the migration of the keratinocytes, of physiological factors such as hormones, cytokines or any molecule capable of influencing their metabolism.

5. Use of the method according to any one of claims 1 to 3 in studying the effect, on the migration of the keratinocytes, of molecules of therapeutic interest of the wound-healing-promoting factor kind.

6. Use of the method according to any one of claims 1 to 3 in studying the effect, on the migration of the keratinocytes, of molecules of cosmetological interest, such as factors protecting against rays, temperature or any physical or chemical condition harmful to the epidermis.

7. Use of the method according to any one of claims 1 to 3 in studying the effect of physical, chemical or biological conditions that may possibly affect the metabolism of the keratinocytes.

8. Use of the method according to any one of claims 1 to 3 in studying the biological state of the keratinocytes in order to diagnose any pathological state, such as a deficiency of wound-healing factor or a state of neoplastic transformation.
